# EUROPEAN PATENT APPLICATION

(11) **EP 3 903 671 A1**
(43) Date of publication of application: **03.11.2021**
(21) Application number: 19902604.8
(22) Date of filing: 09.12.2019
(51) Int. Cl.: A61B 5/02, A61B 5/145

(54) **ELECTRONIC DEVICE**

(30) Priority: 25.12.2018 JP 2018241483
(71) Applicant: Kyocera Corporation, Kyoto-shi, Kyoto 612-8501 (JP)
(72) Inventor: AJIMA Hiromi, Kyoto-shi, Kyoto 612-8501 (JP)
(74) Representative: Viering, Jentschura & Partner mbB Patent- und Rechtsanwälte
(86) International application number: PCT/JP2019/048153
(87) International publication number: WO 2020/137498

(57) **Abstract**

An electronic device includes a sensor capable of detecting pulsation in a target region of a subject, a pressing portion to be pressed toward the target region, and an elastic member interposed between the sensor and the pressing portion.

## Description

### Cross Reference to Related Applications

The present application claims priority to Japanese Patent Application No. 2018-241483 filed in Japan on December 25, 2018, the entire disclosure of which is incorporated herein by reference.

### Technical Field

The present disclosure relates to an electronic device.

### Background Art

In the related art, there is known an electronic device that measures biological information from a target region of a subject, such as a wrist. For example, PTL 1 describes an electronic device wearable on a wrist of a subject to measure the pulse of the subject.

### Citation List

### Patent Literature

PTL 1: Japanese Unexamined Patent Application Publication No. 2002-360530

### Summary of Invention

An electronic device according to an embodiment includes
a sensor capable of detecting pulsation in a target region of a subject,
a pressing portion to be pressed toward the target region, and
an elastic member interposed between the sensor and the pressing portion.

### Brief Description of Drawings

[Fig. 1] Fig. 1 is a diagram illustrating a manner of using an electronic device according to an embodiment.
[Fig. 2] Fig. 2 is a diagram illustrating the electronic device according to the embodiment when viewed from a side.
[Fig. 3] Fig. 3 is a perspective view illustrating the appearance of the front side of the electronic device according to the embodiment.
[Fig. 4] Fig. 4 is a perspective view illustrating the appearance of the back side of the electronic device according to the embodiment.
[Fig. 5] Fig. 5 is a diagram illustrating a cross section of the electronic device according to the embodiment.
[Fig. 6] Fig. 6 is a diagram illustrating a manner of using the electronic device according to the embodiment.
[Fig. 7] Fig. 7 is a functional block diagram illustrating a schematic configuration of the electronic device according to the embodiment.
[Fig. 8] Fig. 8 is a diagram illustrating an example of a pulse wave acquired with a sensor unit.
[Fig. 9] Fig. 9 is a diagram illustrating a time variation in calculated AI.
[Fig. 10] Fig. 10 is a diagram illustrating a calculated AI and a measurement result of blood glucose level.
[Fig. 11] Fig. 11 is a diagram illustrating the relationship between the calculated AI and the blood glucose level.
[Fig. 12] Fig. 12 is a diagram illustrating a calculated AI and a measurement result of triglyceride value.
[Fig. 13] Fig. 13 is a flowchart illustrating a procedure for estimating blood fluidity and the states of glucose metabolism and lipid metabolism.
[Fig. 14] Fig. 14 is a schematic diagram illustrating a schematic configuration of a system according to an embodiment.
[Fig. 15] Fig. 15 is a perspective view illustrating the appearance of the back side of an electronic device according to a modification of the embodiment.
[Fig. 16] Fig. 16 is a diagram illustrating a cross section of an electronic device according to a modification of the embodiment.

### Description of Embodiments

An electronic device capable of easily measuring biological information of a subject can improve its usability. It is an object of the present disclosure to provide an electronic device with high usability. According to the present disclosure, it is possible to provide an electronic device with improved usability. An embodiment will be described hereinafter in detail with reference to the drawings.

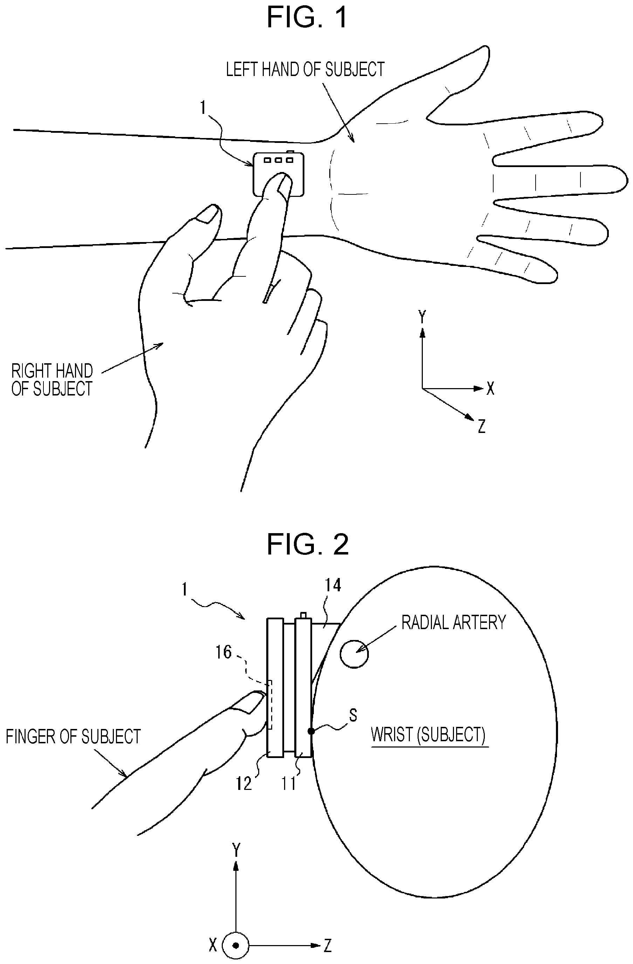

Fig. 1 is a diagram describing a manner of using an electronic device according to an embodiment. That is, Fig. 1 is a diagram illustrating how a subject measures biological information by using an electronic device according to an embodiment.

As illustrated in Fig. 1, an electronic device 1 according to an embodiment is capable of measuring biological information of a subject from, for example, a portion of the subject, such as a wrist of the subject, as a target region. In the example illustrated in Fig. 1, the electronic device 1 remains placed on a target region of the left wrist of the subject. In the example illustrated in Fig. 1, the electronic device 1 remains placed on a target region, the target region being a wrist portion present on the way from the palm toward the elbow of the left hand of the subject.

In the example illustrated in Fig. 1, furthermore, the electronic device 1 is pressed against the target region by the subject with the index finger of the right hand. As illustrated in Fig. 1, the electronic device 1 according to a first embodiment measures biological information of the subject while being pressed against the target region. The finger with which the subject presses the electronic device 1 against the target region is not limited to the index finger of the right hand. The electronic device 1 may be pressed in any manner so long as the electronic device 1 can be pressed against the target region with an appropriate pressing force.

The electronic device 1 can detect pulsation in the target region of the subject upon being placed on the target region. The target region of the subject may be, for example, a region of the body where the ulnar artery or radial artery of the subject is present beneath the skin. The target region of the subject is not limited to a region of the body where the ulnar artery or radial artery of the subject is present beneath the skin, and may be any region of the body where the pulsation of the subject is detectable. Fig. 1 illustrates a state in which the electronic device 1 remains placed on a target region, the target region being a region of the body where the radial artery is located beneath the skin of the wrist of the subject.

The electronic device 1 has a housing whose size may be relatively small in terms of portability and/or ease of measurement or the like, although the size of the housing is not limited. For example, the housing of the electronic device 1 may have a size with four sides equal to 2 cm to 4 cm in the plan view illustrated in Fig. 1. As an example, the housing of the electronic device 1 may have a size with four sides equal to 3.5 cm in the plan view illustrated in Fig. 1. The housing of the electronic device 1 may have a size other than a size with four sides equal to 2 cm to 4 cm. That is, the electronic device 1 may have a size such that the electronic device 1 is housed in a square with sides, each of which is up to 4 cm, in plan view. The housing of the electronic device 1 may be formed into any combination of shapes such as a triangle, a rectangle, any other polygon, a circle, and an ellipse.

Fig. 2 is a diagram illustrating the electronic device 1 illustrated in Fig. 1 when viewed from a side, together with a cross section of the wrist of the subject.

As illustrated in Fig. 2, the electronic device 1 is configured to include a lower housing 11 and an upper housing 12. The lower housing 11 and/or the upper housing 12 may be made of, for example, a material such as ceramic, iron, any other metal, resin, plastic, or aluminum. The lower housing 11 and/or the upper housing 12 may be made of a hard and lightweight material. The material of the lower housing 11 and/or the upper housing 12 is not limited, and may have strength enough to function as a measurement device. Further, the material of the lower housing 11 and/or the upper housing 12 is not excessively heavy and may be relatively light.

As described below, the lower housing 11 and the upper housing 12 can move freely to some extent with respect to each other. That is, in the electronic device 1, even in a state where the lower housing 11 is secured, the upper housing 12 can move freely to some extent. In the electronic device 1, even in a state where the upper housing 12 is secured, the lower housing 11 can move freely to some extent.

As illustrated in Fig. 2, the electronic device 1 includes a pulse contact portion 14 as a portion to be brought into contact with the target region of the subject. The pulse contact portion 14 may be made of, for example, a material such as ceramic, iron, any other metal, resin, plastic, or aluminum. The pulse contact portion 14 may be made of a hard and lightweight material. The material of the pulse contact portion 14 is not limited to any specific one. The material of the pulse contact portion 14 may have strength enough to function as a measurement device and may be relatively lightweight, like the lower housing 11 and/or the upper housing 12.

In one embodiment, the pulse contact portion 14 may be brought into direct or indirect contact with the target region of the subject. As illustrated in Fig. 2, the surface of the wrist of the subject typically has a curved shape. Thus, if a portion of the bottom surface (surface on the Z-axis positive direction side) of the lower housing 11 in the electronic device 1 is brought into contact with the wrist of the subject, the remaining portion of the bottom surface may be floated from the wrist of the subject. Accordingly, as illustrated in Fig. 2, the pulse contact portion 14 may have a wedge-like shape, for example. With this shape, the pulse contact portion 14 can be appropriately brought into abutment against the target region of the subject, with a portion of the bottom surface (for example, a portion S illustrated in Fig. 2) of the lower housing 11 coming into contact with the wrist of the subject. The shape of the pulse contact portion 14 is not limited to a wedge-like shape, and may be any shape that enables the pulse contact portion 14 to appropriately abut against the target region of the subject.

As illustrated in Fig. 2, the upper housing 12 of the electronic device 1 includes a pressing portion 16. The pressing portion 16 represents a portion of the electronic device 1 that is pressed by the subject with a fingertip or the like. That is, the subject or the like sees (or touches) the pressing portion 16, thereby being able to recognize that the pressing portion 16 is to be pressed with a fingertip or the like. As illustrated in Fig. 2, the pressing portion 16 may be formed on the upper surface side of the upper housing 12 (the surface on the Z-axis negative direction side). In the example illustrated in Fig. 2, the pressing portion 16 is formed in a location slightly below the center of the upper surface of the upper housing 12 (in the Y-axis negative direction). However, the pressing portion 16 may be formed in any location in accordance with the manner in which the electronic device 1 measures biological information, such that, for example, the pressing portion 16 is formed substantially at the center of the upper surface of the upper housing 12.

In the example illustrated in Fig. 2, furthermore, the pressing portion 16 is illustrated as a shallow concave portion formed on the upper surface side of the upper housing 12. However, the shape of the pressing portion 16 is not limited to a concave portion. For example, the pressing portion 16 may be formed as a shallow convex portion or the like formed on the upper surface side of the upper housing 12. Alternatively, for example, the pressing portion 16 may merely be a mark painted on the upper surface side of the upper housing 12 (surface on the Z-axis negative direction side) with paint or the like. In the electronic device 1, the pressing portion 16 may be configured in any manner so long as it represents a portion to be pressed by the subject with a fingertip or the like.

The electronic device 1 is placed on the target region such as the wrist of the subject, and the pressing portion 16 is pressed by the subject with a fingertip or the like. As a result, the electronic device 1 is brought into the state illustrated in Fig. 1 during measurement of biological information. When the electronic device 1 is placed on the target region such as the wrist of the subject, the electronic device 1 may be positioned such that the pulse contact portion 14 comes into abutment against the target region of the subject. At this time, the electronic device 1 may be positioned such that, for example, the pulse contact portion 14 comes into abutment against a region of the body where the ulnar artery or radial artery of the subject is present beneath the skin.

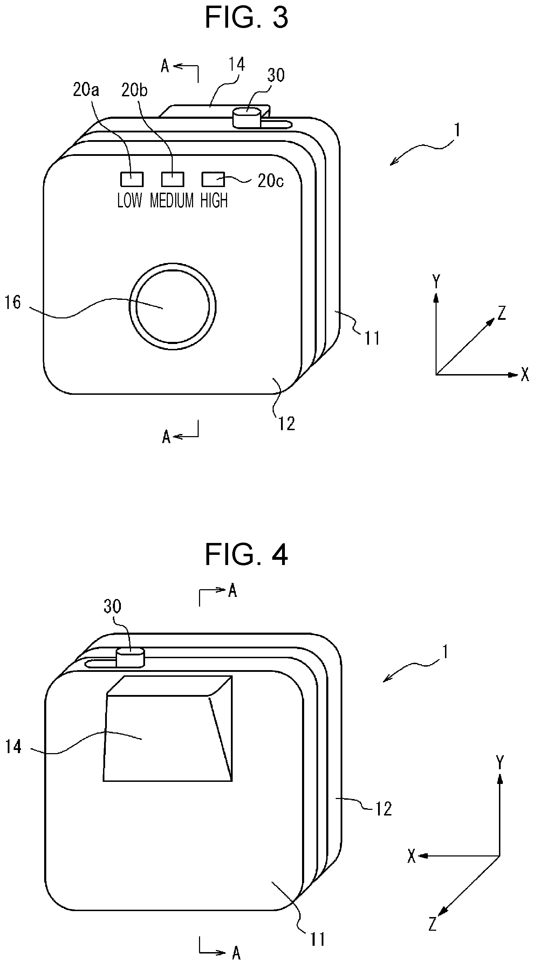

Fig. 3 and Fig. 4 are perspective views illustrating the appearance of the electronic device 1. Fig. 3 is a perspective view illustrating the electronic device 1 illustrated in Fig. 1 when viewed from a viewpoint in the positive direction of the Z axis. Fig. 4 is a perspective view illustrating the electronic device 1 illustrated in Fig. 3 when rotated by 180 degrees about the Y axis.

As illustrated in Fig. 3, the electronic device 1 includes, in appearance, the lower housing 11 and the upper housing 12. As described above, the lower housing 11 includes the pulse contact portion 14, and the upper housing 12 includes the pressing portion 16.

As illustrated in Fig. 3, the electronic device 1 further includes a notification unit 20 and a switch 30.

The notification unit 20 notifies the subject or the like of, for example, information such as a measurement result of biological information. As illustrated in Fig. 3, the notification unit 20 may be, for example, a light-emitting unit such as a light-emitting diode (LED). Alternatively, the notification unit 20 may be a display device such as a liquid crystal display (LCD), an organic EL display (OELD: Organic Electro-Luminescence Display), or an inorganic EL display (IELD: Inorganic Electro-Luminescence Display). Such a display device employed as the notification unit 20 makes it possible to display, for example, relatively detailed information such as the state of glucose metabolism or lipid metabolism of the subject.

Fig. 3 illustrates an example in which the notification unit 20 is constituted by three light-emitting units (20a, 20b, and 20c). In Fig. 3, the notification unit 20a may be, for example, a light-emitting unit indicating that a measurement result of biological information is relatively low. The notification unit 20b may be, for example, a light-emitting unit indicating that a measurement result of biological information is medium (for example, within a permissible range). The notification unit 20c may be, for example, a light-emitting unit indicating that a measurement result of biological information is relatively low. While Fig. 3 illustrates an example in which the notification unit 20 is constituted by three light-emitting units, the notification unit 20 may be constituted by any number of light-emitting units. A light-emitting unit of the notification unit 20 may notify the subject of, for example, information such as a measurement result of biological information in any form such as the color of emitted light, the number of times of blinking, or the like, for example.

The notification unit 20 may notify the subject of not only information such as a measurement result of biological information but also, for example, information such as on/off of the power supply of the electronic device 1 or whether biological information is being measured. At this time, for example, the notification unit 20 may notify the subject of information such as on/off of the power supply of the electronic device 1 or whether biological information is being measured by a different type of light emission from that when notifying the subject of information such as a measurement result of biological information.

In one embodiment, the notification unit 20 may not necessarily be constituted by a light-emitting unit. For example, the notification unit 20 may be constituted by a sound output unit such as a speaker or a buzzer. In this case, the notification unit 20 may notify the subject or the like of, for example, information such as a measurement result of biological information via various sounds, voices, or the like.

In one embodiment, the notification unit 20 may be constituted by, for example, a tactile sensation providing unit such as a vibrator or a piezoelectric element. In this case, the notification unit 20 may notify the subject or the like of, for example, information such as a measurement result of biological information via various types of vibration, tactile sensation feedback, or the like.

The switch 30 may be, for example, a switch that switches on/off of the power supply of the electronic device 1. The switch 30 may be, for example, a switch that causes the electronic device 1 to start measurement of biological information. The switch 30 may be a switch for starting measurement. Fig. 3 illustrates an example in which the switch 30 is constituted by a slide switch. However, the switch 30 may be constituted by any switch such as a push button switch, for example. For example, when the switch 30 is constituted by a push button switch, various operations of the electronic device 1 may be supported in accordance with the number of times the switch 30 is pressed and/or the time during which the switch 30 is pressed, or the like.

As illustrated in Fig. 4, the lower housing 11 of the electronic device 1 includes the pulse contact portion 14. As described above, the pulse contact portion 14 is a member to be appropriately brought into abutment against the target region of the subject when the electronic device 1 measures biological information of the subject. Accordingly, the pulse contact portion 14 may have a size such that, for example, the pulse contact portion 14 is appropriately brought into abutment against a region of the body where the ulnar artery or radial artery of the subject is present beneath the skin. For example, as illustrated in Fig. 4, the pulse contact portion 14 may have a width of about 1 cm to 1.5 cm in the X-axis direction. The pulse contact portion 14 may have a width other than about 1 cm to 1.5 cm in the X-axis direction.

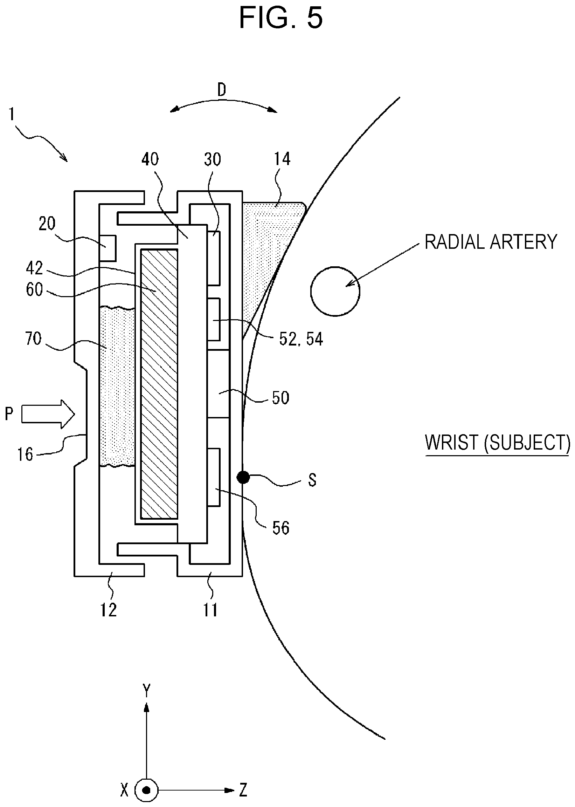

Fig. 5 is a diagram illustrating a cross section of the electronic device 1, together with a cross section of the wrist of the subject. Fig. 5 is a diagram illustrating a cross section taken along line A-A illustrated in Fig. 3 and Fig. 4. The wrist of the subject illustrated in Fig. 5 is similar to that illustrated in Fig. 2.

As illustrated in Fig. 5, the electronic device 1 includes the lower housing 11 and the upper housing 12. The pulse contact portion 14 is disposed on the side of the lower housing 11 closer to the target region. The pulse contact portion 14 comes into abutment against a region of the body where the radial artery of the subject is present beneath the skin. As illustrated in Fig. 5, the lower housing 11 has an opening on the side thereof in the negative direction of the Z axis. The upper housing 12 has an opening on the side thereof in the positive direction of the Z axis. In the example illustrated in Fig. 5, the opening in the lower housing 11 has a smaller size than the opening in the upper housing 12 to allow the lower housing 11 to fit inside the upper housing 12 such that the respective openings face each other. However, the opening in the upper housing 12 may have a smaller size than the opening in the lower housing 11 to allow the upper housing 12 to fit inside the lower housing 11 such that the respective openings face each other. The lower housing 11 and the upper housing 12 may be configured to be movable freely to some extent without interfering with each other.

As illustrated in Fig. 5, a substrate 40 is arranged in the lower housing 11. The notification unit 20 described above is arranged in the upper housing 12.

The substrate 40 may be a typical circuit board on which various electronic components and the like can be arranged. The substrate 40 has a battery holder 42 arranged on the surface thereof on the Z-axis negative direction side. This battery holder is a member for securing a battery 60. The battery 60 may be any power supply, for example, a button battery (coin battery) such as CR2032. Alternatively, the battery 60 may be, for example, a rechargeable storage battery. The battery 60 may include, for example, a lithium-ion battery and a control circuit or the like for charging and discharging the lithium-ion battery, if necessary. The battery 60 may supply power to the functional units of the electronic device 1.

Various electronic components may be arranged on the surfaces of the substrate 40 on the Z-axis negative and positive direction sides. In the example illustrated in Fig. 5, the switch 30, a sensor 50, a control unit 52, a storage unit 54, and a communication unit 56 are arranged on the surfaces of the substrate 40 on the Z-axis negative and positive direction sides.

The sensor 50 includes, for example, an angular speed sensor and detects pulsation from the target region to acquire a pulse wave. The sensor 50 may detect a displacement of the pulse contact portion 14 based on the pulse wave of the subject. The sensor 50 may be, for example, an acceleration sensor or may be a sensor such as a gyro sensor. Alternatively, the sensor 50 may be an angular speed sensor. The sensor 50 will further be described below.

As illustrated in Fig. 5, the sensor 50 is secured to the substrate 40. The substrate 40 is secured within the lower housing 11. The pulse contact portion 14 is secured to the outside of the lower housing 11. Thus, a movement of the pulse contact portion 14 is transmitted to the sensor 50 through the lower housing 11 and the substrate 40. Accordingly, the sensor 50 can detect the pulsation in the target region of the subject through the pulse contact portion 14, the lower housing 11, and the substrate 40.

In the example illustrated in Fig. 5, the sensor 50 is arranged in such a manner as to be in contact with the inside of the lower housing 11. However, in one embodiment, the sensor 50 may be arranged in such a manner as not to be in contact with the inside of the lower housing 11. For example, the sensor 50 may have any configuration in which a movement of at least one of the pulse contact portion 14, the lower housing 11, and the substrate 40 is transmitted to the sensor 50.

The control unit 52 is a processor that controls and manages the entire electronic device 1, including the functional blocks of the electronic device 1. Further, the control unit 52 is a processor that calculates, from the acquired pulse wave, an index based on the propagation phenomenon of the pulse wave. The control unit 52 is constituted by a processor such as a CPU (Central Processing Unit) that executes a program specifying a control procedure and a program for calculating an index based on the propagation phenomenon of the pulse wave, and the programs are stored in a storage medium, such as the storage unit 54, for example. Further, the control unit 52 estimates a state related to glucose metabolism, lipid metabolism, or the like of the subject on the basis of the calculated index. The control unit 52 sends data to the notification unit 20.

The storage unit 54 stores programs and data. The storage unit 54 may include any non-transitory storage medium such as a semiconductor storage medium and a magnetic storage medium. The storage unit 54 may include a plurality of types of storage media. The storage unit 54 may include a combination of a portable storage medium, such as a memory card, an optical disk, or a magneto-optical disk, and a storage medium reading device. The storage unit 54 may include a storage device used as a temporary storage area such as a RAM (Random Access Memory). The storage unit 54 stores various types of information and/or programs for operating the electronic device 1, and also functions as a work memory. The storage unit 54 may store, for example, a measurement result of the pulse wave acquired by the sensor 50.

The communication unit 56 performs wired communication or wireless communication with an external device to transmit and receive various data. The communication unit 56 communicates with, for example, an external device that stores biological information of the subject to manage the health condition, and transmits the measurement result of the pulse wave measured by the electronic device 1 and/or the health condition estimated by the electronic device 1 to the external device. The communication unit 56 may be, for example, a communication module that supports Bluetooth (registered trademark), Wi-Fi, or the like.

The arrangement of the switch 30, the sensor 50, the control unit 52, the storage unit 54, and the communication unit 56 is not limited to that in the example illustrated in Fig. 5. For example, the functional units described above may be arranged at any positions on the substrate 40. The functional units described above may not necessarily be arranged on the same surface of the substrate 40, and may be each arranged on either side of the substrate 40, as necessary. The arrangement of the notification unit 20 is not also limited to that in the example illustrated in Fig. 5. For example, the notification unit 20 may not be arranged in the upper housing 12, but may be arranged on the substrate 40 or the battery holder 42 or in the lower housing 11. In this case, for example, the upper housing 12 may be perforated at any position such that, for example, light emitted from the notification unit 20 serving as a light-emitting unit can be visually recognized. Alternatively, for example, the lower housing 11 or the upper housing 12 may be provided with a light guide plate at any position such that, for example, light emitted from the notification unit 20 serving as a light-emitting unit can be visually recognized even from outside the lower housing 11 or the upper housing 12.

In a case where the electronic device 1 is connected to an external device in a wired or wireless manner, for example, at least some of the functional units such as the notification unit 20, the switch 30, the control unit 52, the storage unit 54, and the communication unit 56 may be included in the external device, as necessary.

As illustrated in Fig. 5, in the electronic device 1, the lower housing 11 and the upper housing 12 are connected to each other through an elastic member 70. In the example illustrated in Fig. 5, the upper housing 12 and the battery holder 42 are connected to each other through the elastic member 70. However, the elastic member 70 may connect, for example, the upper housing 12 and the substrate 40 or the lower housing 11 to each other. In one embodiment, the elastic member 70 may connect any member on the lower housing 11 side and any member on the upper housing 12 side to each other. The elastic member 70 may be an elastic member deformable along at least any one axis among three axes orthogonal to one another (for example, the Y axis, the Y axis, and the Z axis). The elastic member 70 is a three-dimensionally deformable member.

The elastic member 70 may be configured to include any elastic body having appropriate elasticity, such as a spring, a resin, or a sponge, for example. The elastic member 70 may be formed by, for example, a silicone sheet of a predetermined thickness having predetermined elasticity. The elastic member 70 will further be described below. The elastic member 70 may be bonded to the upper housing 12 using an adhesive, a double-sided adhesive tape, or the like. The elastic member 70 may be bonded to the lower housing 11, the substrate 40, or the battery holder 42 using an adhesive, a double-sided adhesive tape, or the like. The elastic member 70 may be bonded to any other member such that the influence on the deformation of the elastic member 70 can be reduced. That is, the elastic member 70 may be configured to be appropriately deformable even when the elastic member 70 is bonded to any other member.

As described above, the electronic device 1 according to an embodiment includes the pressing portion 16, the sensor 50, and the elastic member 70. The pressing portion 16 is pressed toward the target region of the subject. In Fig. 5, the subject may press the pressing portion 16 in, for example, a direction indicated by an arrow P. The sensor 50 detects pulsation in the target region of the subject. The elastic member 70 is interposed between the sensor 50 and the pressing portion 16.

As illustrated in Fig. 5, in a state where the electronic device 1 remains placed on the target region of the subject, the pulse contact portion 14 is in contact with the target region of the subject, that is, the skin over the radial artery of the subject. The pressing portion 16 is pressed toward the target region, that is, in the direction indicated by the arrow P by the subject with, for example, a finger of the right hand or the like. Due to the elastic force of the elastic body 140 arranged between the pressing portion 16 (the upper housing 12) and the sensor 50, the sensor 50 is urged toward the target region of the subject (together with the lower housing 11 and the pulse contact portion 14). The pulse contact portion 14, which is urged by the elastic force of the elastic body 140, is in contact with the skin over the radial artery of the subject. In this case, the pulse contact portion 14 is displaced in accordance with the movement of the radial artery of the subject, that is, the pulsation. Accordingly, the sensor 50, which operates in association with the pulse contact portion 14, is also displaced in accordance with the movement of the radial artery of the subject, that is, the pulsation. For example, as illustrated in Fig. 5, the displacement about an axis S can occur in a direction indicated by an arrow D, with the pressing portion 16 pressed in the direction indicated by the arrow P by the subject. The axis S may be a portion where the bottom surface of the lower housing 11 contacts the wrist of the subject. In this case, the position pressed in the direction indicated by the arrow P (i.e., the position of the pressing portion 16) may be a position between the axis S and the pulse contact portion 14 (target region) in the XY plane.

In this embodiment, the sensor 50, which operates in association with the pulse contact portion 14, is coupled to the upper housing 12 (the pressing portion 16) through the elastic member 70. Thus, the sensor 50 is given a somewhat free range of motion because of the flexibility of the elastic member 70. The flexibility of the elastic member 70 further makes it difficult to hinder the movement of the sensor 50. The elastic member 70 having appropriate elasticity deforms in accordance with the pulsation in the target region of the subject. In the electronic device 1 according to this embodiment, therefore, the sensor 50 can sensitively detect the pulsation in the target region of the subject. In addition, the electronic device 1 according to this embodiment is displaced in accordance with the pulse wave, which can eliminate the congestion of the subject and eliminate the pain of the subject. In this manner, in this embodiment, the elastic member 70 may be deformable in accordance with the pulsation in the target region of the subject. Further, the elastic member 70 may be elastically deformed to such an extent that the pulsation in the target region of the subject is detectable by the sensor 50.

As described above, the electronic device 1 according to an embodiment can function as a small and lightweight measurement device. The electronic device 1 according to an embodiment is not only excellent in portability but also capable of extremely easily measuring biological information of the subject. In addition, the electronic device 1 according to an embodiment can measure biological information alone, without cooperating with any other external device or the like. In this case, there is no need to carry any other accessory such as a cable. The electronic device 1 according to an embodiment can therefore increase usability.

In this embodiment, the sensor 50 may be, for example, a sensor that detects, for each of a plurality of axes, at least one of the angle (inclination), angular speed, and angular acceleration of an object, such as a gyro sensor (gyroscope). In this case, the sensor 50 can detect complex motion based on the pulsation in the target region of the subject as the respective parameters for the plurality of axes. Alternatively, the sensor 50 may be a six-axis sensor that is a combination of a three-axis gyro sensor and a three-axis acceleration sensor.

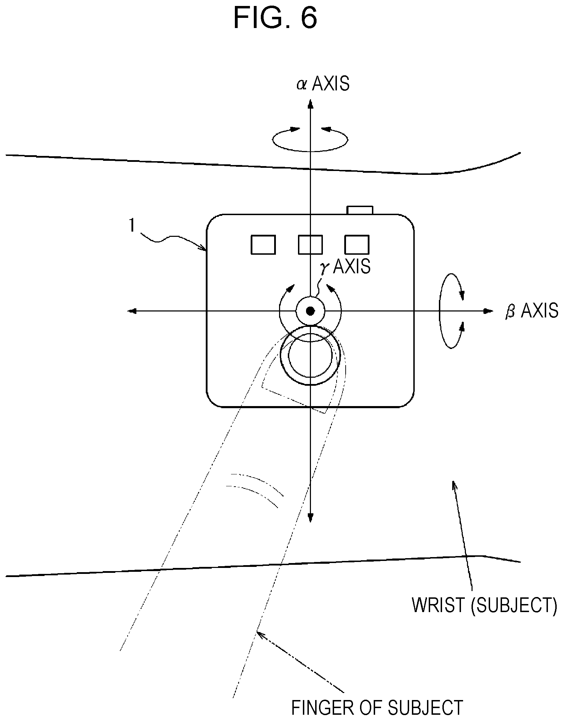

Fig. 6 is a diagram illustrating an example manner of using the electronic device 1. Fig. 6 is a diagram illustrating an enlarged version of the situation illustrated in Fig. 1.

For example, as illustrated in Fig. 6, the sensor 50 built in the electronic device 1 may detect a rotational movement about each of three axes, namely, an α axis, a β axis, and a γ axis. The α axis may be, for example, an axis extending in a direction substantially orthogonal to the radial artery of the subject. The β axis may be, for example, an axis extending in a direction substantially parallel to the radial artery of the subject. The γ axis may be, for example, an axis extending in a direction substantially orthogonal to both the α axis and the β axis.

In this embodiment, accordingly, the sensor 50 may detect pulsation in the target region of the subject as a portion of a rotational movement about a predetermined axis. Alternatively, the sensor 50 may detect pulsation in the target region of the subject as rotational movements on at least two axes or as rotational movements on three axes. In the present disclosure, the "rotational movement" may not necessarily be a movement including a displacement along a circular orbit by one or more turns. For example, in the present disclosure, the rotational movement may be, for example, a partial displacement along a circular orbit by less than one turn (for example, a displacement along an arc) .

As illustrated in Fig. 6, the electronic device 1 according to this embodiment can detect, for example, respective rotational movements about three axes using the sensor 50. The electronic device 1 according to this embodiment combines the plurality of results detected by the sensor 50 by, for example, adding them up, and can thus increase the detection sensitivity of the pulse wave of the subject. The computation, such as adding up, may be performed by the control unit 52, for example. In this case, the control unit 52 may calculate the index of the pulse wave based on the pulsation detected by the sensor 50.

For example, in the example illustrated in Fig. 6, the changes in signal strength with time based on the rotational movements of the sensor 50 about the α axis and the β axis have remarkable peaks based on the pulse wave of the subject. Thus, for example, the control unit 52 adds up the detection results for the α axis, the β axis, and the γ axis, and can thus increase the detection accuracy of the pulse wave of the subject. The electronic device 1 according to this embodiment can therefore improve the usefulness when the subject measures the pulse wave.

In one embodiment, the control unit 52 of the electronic device 1 may calculate the index of the pulse wave based on the pulsation detected by the sensor 50. In this case, the control unit 52 may combine (for example, add up) the results detected by the sensor 50 as rotational movements on at least two axes (for example, rotational movements on three axes). The electronic device 1 according to this embodiment can detect pulse wave signals of a plurality of directions. Thus, the electronic device 1 according to this embodiment combines detection results for a plurality of axes, thereby increasing the signal strength compared to a detection result for a single axis. The electronic device 1 according to this embodiment can therefore detect a signal having a good SN ratio and increase the detection sensitivity, making it possible to achieve stable measurement.

In the detection result for the γ axis illustrated in Fig. 6, the peak based on the pulse wave of the subject is expected not to appear more noticeably than that in the detection result for the remaining α axis or β axis. In this manner, adding a detection result having a low signal level, such as the detection result for the γ axis, to a detection result for another axis may result in a reduction in SN ratio. In addition, a detection result having a low signal level may be mostly regarded as a noise component. In this case, the detection result having a low signal level may not contain a satisfactory pulse wave component. In this embodiment, accordingly, if there is an axis for which the detection result is less than a predetermined threshold among the detection results for the plurality of axes, the control unit 52 may not add the detection result for the axis.

For example, it is assumed that the pulsation of a certain subject is detected by the sensor 50 as the respective rotational movements about the α axis, the β axis, and the γ axis. As a result, the peak values in the detection results for the α axis, the β axis, and the γ axis are assumed to exceed the predetermined threshold. In this case, the control unit 52 may add up all of the detection result for the α axis, the detection result for the β axis, and the detection result for the γ axis to calculate the sum as the index of the pulse wave based on the pulsation detected by the sensor 50.

On the other hand, for example, as a result of detecting the pulsation of a certain subject, the peak values in the detection results for the α axis and the β axis are assumed to exceed the predetermined threshold. However, the peak value in the detection result for the γ axis is assumed not to exceed the predetermined threshold. In this case, the control unit 52 may add up only the detection results for the α axis and the β axis to calculate the sum as the index of the pulse wave based on the pulsation detected by the sensor 50.

When performing such processing, the control unit 52 may set thresholds, which are used as a reference to determine whether the detection results for the respective axes are to be added up, to be separate for the respective axes or to be the same for the respective axes. In both cases, a threshold may be set appropriately so that the pulsation of the subject can be suitably detected in a detection result for each axis.

In this manner, in the electronic device 1 according to this embodiment, the control unit 52 may combine only results having components greater than or equal to a predetermined threshold among the results detected by the sensor 50 as rotational movements on at least two axes. Thus, the electronic device 1 according to this embodiment can suppress the reduction in the SN ratio of a detection result. The electronic device 1 according to this embodiment can therefore improve the usefulness when the subject measures the pulse wave.

As described above, when adding up detection results for a plurality of axes, merely adding up the detection results for the respective axes may cause a problem. This is presumably because the results detected by the sensor 50 do not match in polarity depending on the positional relationship between the direction of the pulsation of the subject and the sensor 50. For example, a detection result for a certain axis may be reversed in polarity between when the pulsation of the right hand of the subject is detected and when the pulsation of the left hand of the subject is detected using the sensor 50.

For example, when the pulsation of the subject is detected, it is assumed that an upward peak is approximately periodically detected for a detection result for a certain axis. However, it is also assumed that a downward peak is approximately periodically detected for a detection result for another axis. In this manner, when detection results for a plurality of axes are reversed in polarity, merely adding up the detection results may cause the peaks to be canceled out each other, and a satisfactory result may not be obtained.

In this embodiment, accordingly, when detection results for a plurality of axes are reversed in polarity, the control unit 52 may invert the polarity of the detection result for at least one axis before adding the detection result to the detection results for the other axes. For example, if detection results for two axes are reversed in polarity, the control unit 52 may invert the polarity of the detection result for one axis in accordance with the other axis.

In this manner, in the electronic device 1 according to this embodiment, the control unit 52 may combine the results detected by the sensor 50 as rotational movements on at least two axes after the polarities of the results are made to match each other. The electronic device 1 according to this embodiment can increase the detection accuracy of the pulse wave of the subject. The electronic device 1 according to this embodiment can therefore improve the usefulness when the subject measures the pulse wave.

As described above, when processing for matching the polarities of detection results for a plurality of axes is performed by inverting the polarity of the detection result for at least one axis, it is necessary to determine the directions of the polarities in the respective detection results. The determination of the polarity directions can be performed by various methods. For example, the control unit 52 may determine whether the peak of the detection result for each axis is directed to the positive direction side or the negative direction side of the signal strength. Alternatively, for example, the control unit 52 may determine whether the peak of the detection result for each axis is larger or smaller than the average value of the signal. In order to invert the polarity of the detection result for at least one axis, the control unit 52 may multiply the detection result whose polarity is to be inverted by minus 1.

Further, after appropriately inverting the polarity of a detection result in the way described above, the control unit 52 may add or subtract a predetermined value to or from the entire detection result and then add the detection result to the detection results for the other axes. Alternatively, before adding up the detection results for the plurality of axes, the control unit 52 may appropriately weight the detection results for the respective axes or appropriately correct the detection results for the respective axes.

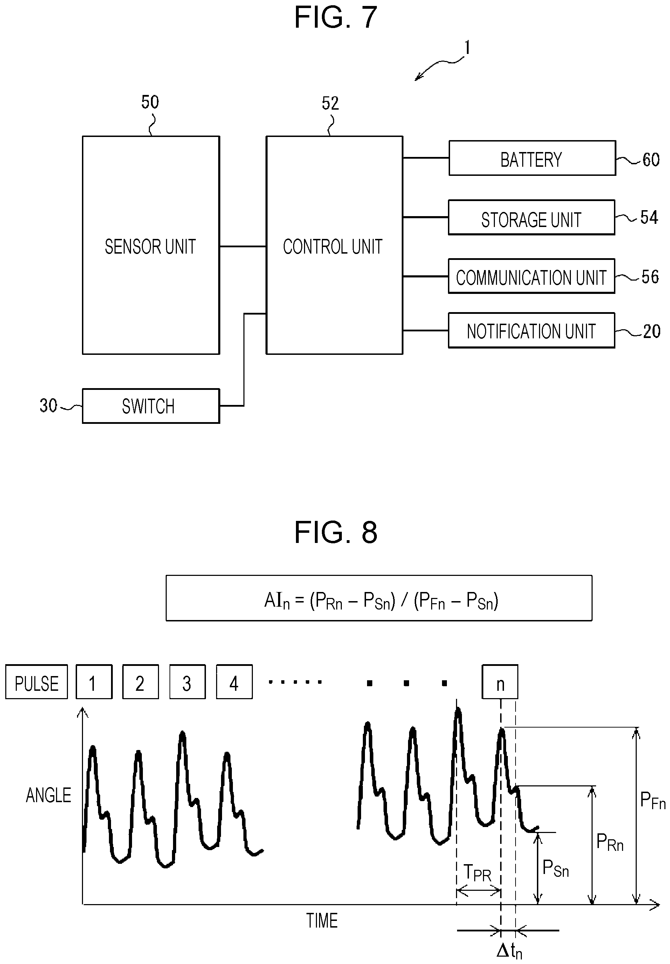

Fig. 7 is a functional block diagram illustrating a schematic configuration of the electronic device 1. The electronic device 1 includes the notification unit 20, the switch 30, the sensor 50, the control unit 52, the storage unit 54, the communication unit 56, and the battery 60. These functional units have been described above.

Fig. 8 is a diagram illustrating an example of a pulse wave acquired at the wrist using the electronic device 1. Fig. 8 illustrates a case where an angular speed sensor is used as the sensor 50 that senses pulsation. Fig. 8 illustrates that an angular speed acquired by the angular speed sensor is integrated with respect to time, in which the horizontal axis represents time, and the vertical axis represents angle. The acquired pulse wave may contain noise caused by, for example, body movement of the subject and may thus be corrected by a filter that removes DC (Direct Current) components to extract only the pulsation components.

A method for calculating the index based on the pulse wave from the acquired pulse wave will be described with reference to Fig. 8. The propagation of the pulse wave is a phenomenon in which a heartbeat caused by blood pumped out of the heart is transmitted through the wall of an artery or the blood. The heartbeat caused by blood pumped out of the heart reaches the periphery of limbs as a forward traveling wave, and a portion thereof is reflected by a blood vessel branch portion, a blood-vessel-diameter changing portion, or the like and returns as a reflected wave. The index based on the pulse wave is, for example, the pulse wave velocity PWV of the forward traveling wave, the magnitude PR of the reflected wave of the pulse wave, the time difference Δt between the forward traveling wave and reflected wave of the pulse wave, the AI (Augmentation Index), which is represented by the ratio of the magnitudes of the forward traveling wave and reflected wave of the pulse wave, or the like.

The pulse wave illustrated in Fig. 8 is a pulse wave with n pulses of a user, where n is an integer greater than or equal to 1. The pulse wave is a composite wave in which a forward traveling wave generated by the ejection of blood from the heart and a reflected wave generated from the blood vessel branch or the blood-vessel-diameter changing portion overlap each other. In Fig. 8, the magnitude of the peak of the pulse wave resulting from the forward traveling wave for each pulse is denoted by P_{Fn}, the magnitude of the peak of the pulse wave resulting from the reflected wave for each pulse is denoted by P_{Rn}, and the minimum value of the pulse wave of each pulse is denoted by P_{Sn}. In Fig. 8, the interval between the peaks of pulses is denoted by T_{PR}.

The index based on the pulse wave is obtained by quantifying information obtained from the pulse wave. For example, the PWV, which is one index based on the pulse wave, is calculated based on the difference in propagation time between pulse waves measured at two target regions such as an upper arm and an ankle and the distance between the two target regions. Specifically, the PWV is calculated by acquiring pulse waves at two points along an artery (for example, an upper arm and an ankle) in synchronization with each other and dividing a distance difference (L) between the two points by a time difference (PTT) between the pulse waves at the two points. For example, as the magnitude P_{R} of the reflected wave, which is one index based on the pulse wave, the magnitude P_{Rn} of a peak of the pulse wave resulting from the reflected wave may be calculated, or P_{Rave} obtained by averaging the n magnitudes may be calculated. For example, as the time difference Δt between the forward traveling wave and reflected wave of the pulse wave, which is one index based on the pulse wave, a time difference Δtₙ in a predetermined pulse may be calculated, or Δtₐᵥₑ obtained by averaging the n time differences may be calculated. For example, the AI, which is one index based on the pulse wave, is obtained by dividing the magnitude of the reflected wave by the magnitude of the forward traveling wave, and is expressed by AIₙ = (P_{Rn} - P_{Sn})/(P_{Fn} - P_{Sn}). AIₙ is the AI for each pulse. The AI may be obtained by, for example, measuring a pulse wave for several seconds, calculating an average value AIₐᵥₑ of AIₙ (n is an integer of 1 to n) for the respective pulses, and setting the average value AIₐᵥₑ as an index based on the pulse wave.

The pulse wave velocity PWV, the magnitude P_{R} of the reflected wave, the time difference Δt between the forward traveling wave and the reflected wave, and the AI change depending on the stiffness of the blood vessel wall, and can thus be used to estimate the state of arteriosclerosis. For example, if the blood vessel wall is stiff, the pulse wave velocity PWV is large. For example, if the blood vessel wall is stiff, the magnitude P_{R} of the reflected wave is large. For example, if the blood vessel wall is stiff, the time difference Δt between the forward traveling wave and the reflected wave is small. For example, if the blood vessel wall is stiff, the AI is large. The electronic device 1 can, in addition to estimating the state of arteriosclerosis, estimate blood fluidity (viscosity) using these indices based on the pulse wave. In particular, the electronic device 1 can estimate a change in blood fluidity from a change in the index based on the pulse wave acquired from the same target region of the same subject in a period during which the state of arteriosclerosis does not substantially change (for example, within several days). The blood fluidity represents a measure of the ease of blood flow. For example, if the blood fluidity is low, the pulse wave velocity PWV is small. For example, if the blood fluidity is low, the magnitude P_{R} of the reflected wave is small. For example, if the blood fluidity is low, the time difference Δt between the forward traveling wave and the reflected wave is large. For example, if the blood fluidity is low, the AI is small.

While this embodiment presents an example in which the electronic device 1 calculates the pulse wave velocity PWV, the magnitude P_{R} of the reflected wave, the time difference Δt between the forward traveling wave and the reflected wave, and the AI as example indices based on the pulse wave, the indices based on the pulse wave are not limited thereto. For example, the electronic device 1 may use the posterior systolic blood pressure as an index based on the pulse wave.

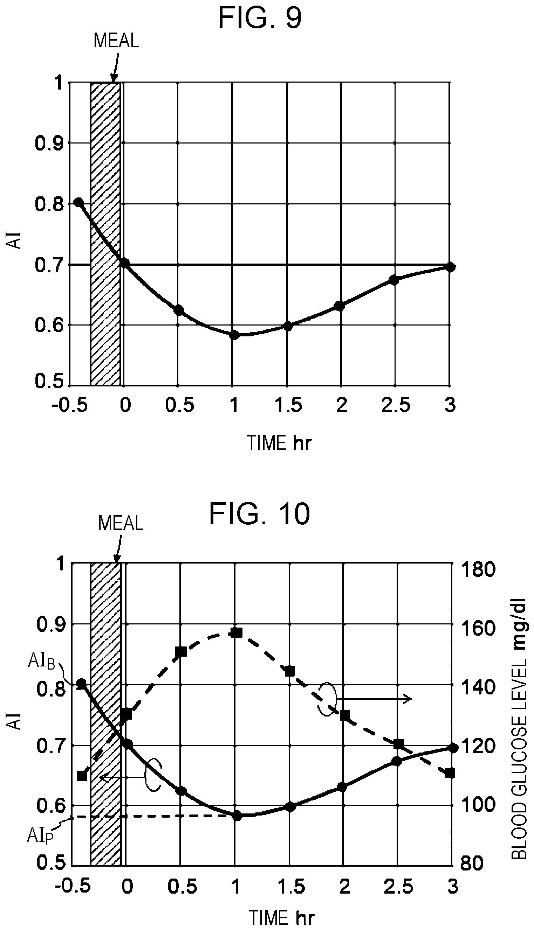

Fig. 9 is a diagram illustrating a time variation in calculated AI. In this embodiment, the pulse wave was acquired for about five seconds using the electronic device 1 including an angular speed sensor 131. The control unit 52 calculated the AI for each pulse from the acquired pulse wave, and further calculated the average value AIₐᵥₑ of these AIs. In this embodiment, the electronic device 1 acquired pulse waves at a plurality of timings before and after a meal, and calculated an average value of the AIs (hereinafter referred to as the AI) as an example index based on the acquired pulse waves. In Fig. 9, the horizontal axis represents the passage of time, with the first measurement time after the meal being 0. In Fig. 9, the vertical axis represents the AI calculated from the pulse wave acquired at that time. The pulse waves were acquired over the radial artery while the subject remained at rest.

The electronic device 1 acquired pulse waves every 30 minutes before the meal, immediately after the meal, and after the meal, and calculated a plurality of AIs on the basis of the respective pulse waves. The AI calculated from the pulse wave acquired before the meal was about 0.8. The AI immediately after the meal became smaller than that before the meal, and the AI reached the minimum extreme value at about 1 hour after the meal. The AI gradually increased until the measurement was finished at 3 hours after the meal.

The electronic device 1 can estimate a change in blood fluidity from the change in calculated AI. For example, if red blood cells, white blood cells, and platelets in blood are aggregated together or adhesion increases, blood fluidity decreases. For example, if the water content of plasma in blood becomes low, blood fluidity decreases. These changes in blood fluidity are caused by, for example, the glycolipid state described below or the health condition of the subject, such as heatstroke, dehydration, or hypothermia. Before the health condition of the subject becomes serious, the subject can recognize a change in their blood fluidity by using the electronic device 1 of this embodiment. From the change in AI before and after the meal illustrated in Fig. 9, it can be estimated that the blood fluidity decreased after the meal, the blood fluidity decreased to the lowest level at about 1 hour after the meal, and then the blood fluidity gradually increased. The electronic device 1 may notify the subject of blood fluidity by expressing "thick" for a low blood fluidity state and "thin" for a high blood fluidity state. For example, the electronic device 1 may determine whether the blood is "thick" or "thin" on the basis of the average value of AIs for the age of the subject. The electronic device 1 may determine that the blood is "thin" if the calculated AI is larger than the average value, and determine that the blood is "thick" if the calculated AI is smaller than the average value. The electronic device 1 may determine whether the blood is "thick" or "thin" on the basis of, for example, the AI before the meal. The electronic device 1 may compare the AI after the meal with the AI before the meal and estimate the degree to which the blood is "thick". The electronic device 1 can use, for example, the AI before the meal, that is, the AI on an empty stomach, as an index for the vascular age (vascular stiffness) of the subject. For example, the electronic device 1 calculates an amount of change in calculated AI on the basis of the AI of the subject before the meal, that is, the AI on an empty stomach, thereby making it possible to reduce an estimation error caused by the vascular age (vascular stiffness) of the subject. It is therefore possible to more accurately estimate a change in blood fluidity.

Fig. 10 is a diagram illustrating a calculated AI and a measurement result of blood glucose level. The pulse wave acquisition method and the AI calculation method are the same as those in the embodiment illustrated in Fig. 9. In Fig. 10, the right vertical axis represents blood glucose level in blood, and the left vertical axis represents calculated AI. In Fig. 10, the solid line indicates an AI calculated from an acquired pulse wave, and the dotted line indicates a measured blood glucose level. The blood glucose level was measured immediately after the acquisition of the pulse wave. The blood glucose level was measured using the blood glucose meter "Medisafe Fit", manufactured by Terumo Corporation. Compared to the blood glucose level before the meal, the blood glucose level immediately after the meal increased by about 20 mg/dl. The blood glucose level reached the maximum extreme value at about 1 hour after the meal. Thereafter, the blood glucose level gradually decreased until the measurement was finished, and became almost the same as the blood glucose level before the meal at about 3 hours after the meal.

As illustrated in Fig. 10, the blood glucose level before and after a meal has a negative correlation with the AI calculated from the pulse wave. As the blood glucose level increases, glucose in blood causes aggregation of red blood cells and platelets or increases adhesion, and, as a result, blood fluidity may decrease. A decrease in blood fluidity may decrease the pulse wave velocity PWV. A decrease in pulse wave velocity PWV may increase the time difference Δt between the forward traveling wave and the reflected wave. An increase in the time difference Δt between the forward traveling wave and the reflected wave may cause the magnitude P_{R} of the reflected wave to decrease compared to the magnitude P_{F} of the forward traveling wave. A decrease in the magnitude P_{R} of the reflected wave compared to the magnitude P_{F} of the forward traveling wave may decrease the AI. Since the AI within several hours (in this embodiment, 3 hours) after the meal has a correlation with the blood glucose level, the variation in the blood glucose level of the subject can be estimated from the variation in AI. If the blood glucose level of the subject is measured in advance and the correlation with the AI is acquired, the electronic device 1 can estimate the blood glucose level of the subject from the calculated AI.

The electronic device 1 can estimate the state of glucose metabolism of the subject on the basis of the time of occurrence of the minimum extreme value of the AI detected for the first time after the meal, namely, AI_{P}. The electronic device 1 estimates, for example, the blood glucose level as the state of glucose metabolism. In an example estimation of the state of glucose metabolism, for example, if the minimum extreme value AI_{P} of the AI detected for the first time after the meal is detected after a lapse of a predetermined time or longer (for example, about 1.5 hours or longer after the meal), the electronic device 1 can estimate that the subject has a glucose metabolism disorder (patient with diabetes).

The electronic device 1 can estimate the state of glucose metabolism of the subject on the basis of the difference (AI_{B} - AI_{P}) between AI_{B}, which is the AI before the meal, and AI_{P}, which is the minimum extreme value of the AI detected for the first time after the meal. In an example estimation of the state of glucose metabolism, for example, if (AI_{B} - AI_{P}) is greater than or equal to a predetermined value (for example, greater than or equal to 0.5), it can be estimated that the subject has a glucose metabolism disorder (patient with postprandial hyperglycemia).

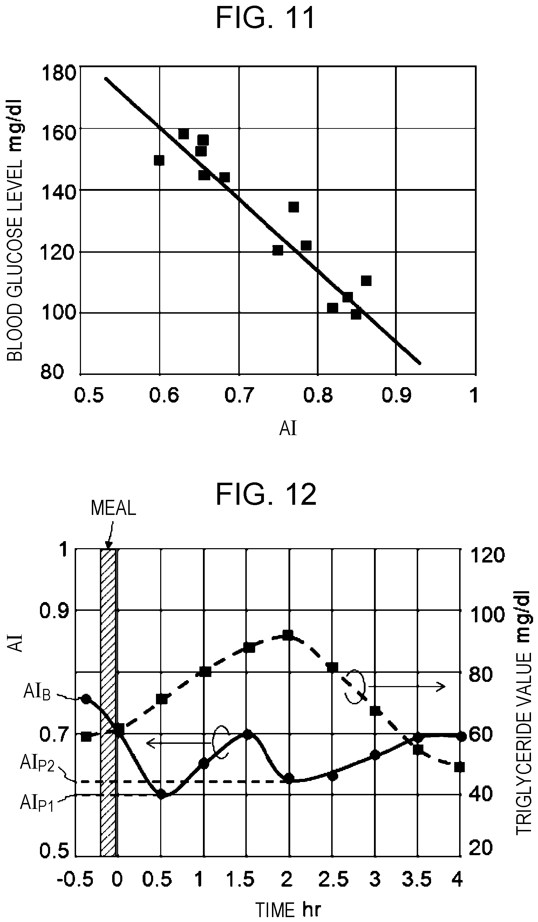

Fig. 11 is a diagram illustrating the relationship between the calculated AI and the blood glucose level. The calculated AI and the blood glucose level were acquired within 1 hour after the meal, within which the blood glucose level varies greatly. The data in Fig. 11 includes a plurality of different pieces of data after the meal for the same subject. As illustrated in Fig. 11, the calculated AI and the blood glucose level exhibited a negative correlation. The correlation coefficient between the calculated AI and the blood glucose level was greater than or equal to 0.9 and exhibited a very high correlation. For example, the correlation between the calculated AI and the blood glucose level illustrated in Fig. 11 may be acquired for each subject in advance, thus allowing the electronic device 1 to estimate the blood glucose level of the subject from the calculated AI.

Fig. 12 is a diagram illustrating a calculated AI and a measurement result of triglyceride value. The pulse wave acquisition method and the AI calculation method are the same as those in the embodiment illustrated in Fig. 9. In Fig. 12, the right vertical axis represents triglyceride value in blood, and the left vertical axis represents AI. In Fig. 12, the solid line indicates an AI calculated from an acquired pulse wave, and the dotted line indicates a measured triglyceride value. The triglyceride value was measured immediately after the acquisition of the pulse wave. The triglyceride value was measured using the lipid measurement device "Pocket Lipid", manufactured by Techno Medica Co., Ltd. Compared to the triglyceride value before the meal, the maximum extreme value of the triglyceride value after the meal increased by about 30 mg/dl. The triglyceride reached the maximum extreme value at about 2 hours after the meal. Thereafter, the triglyceride value gradually decreased until the measurement was finished, and became almost the same as the triglyceride value before the meal at about 3.5 hours after the meal.

In contrast, regarding minimum extreme values of the calculated AI, a first minimum extreme value AI_{P1} was detected at about 30 minutes after the meal, and a second minimum extreme value AI_{P2} was detected at about 2 hours after the meal. The first minimum extreme value AI_{P1} detected at about 30 minutes after the meal can be estimated to be caused by the influence of the blood glucose level after the meal described above. The time of occurrence of the second minimum extreme value AI_{P2} detected at about 2 hours after the meal substantially matches the time of occurrence of the maximum extreme value of the triglyceride detected at about 2 hours after the meal. From this, it can be estimated that the second minimum extreme value AI_{P2} detected after a predetermined time or longer from the meal is caused by the influence of triglyceride. Like the blood glucose level, it was found that the triglyceride value before and after a meal had a negative correlation with the AI calculated from the pulse wave. In particular, since the minimum extreme value AI_{P2} of the AI, which is detected after a predetermined time or longer (in this embodiment, about 1.5 hours or longer) from the meal, has a correlation with the triglyceride value, the variation in the triglyceride value of the subject can be estimated from the variation in AI. If the triglyceride value of the subject is measured in advance and the correlation with the AI is acquired, the electronic device 1 can estimate the triglyceride value of the subject from the calculated AI.

The electronic device 1 can estimate the state of lipid metabolism of the subject on the basis of the time of occurrence of the second minimum extreme value AI_{P2} detected after the predetermined time or longer after the meal. The electronic device 1 estimates, for example, a lipid value as the state of lipid metabolism. In an example estimation of the state of lipid metabolism, for example, if the second minimum extreme value AI_{P2} is detected after a lapse of a predetermined time or longer (for example, 4 hours or longer) after the meal, the electronic device 1 can estimate that the subject has a lipid metabolism disorder (patient with hyperlipidemia).

The electronic device 1 can estimate the state of lipid metabolism of the subject on the basis of the difference (AI_{B} ― AI_{P2}) between AI_{B}, which is the AI before the meal, and the second minimum extreme value AIP2 detected after the predetermined time or longer after the meal. In an example estimation of lipid metabolism disorder, for example, if (AI_{B} ― AI_{P2}) is greater than or equal to 0.5, the electronic device 1 can estimate that the subject has a lipid metabolism disorder (patient with postprandial hyperlipidemia).

From the measurement results illustrated in Fig. 10 to Fig. 12, the electronic device 1 of this embodiment can estimate the state of glucose metabolism of the subject on the basis of the first minimum extreme value AI_{P1} detected earliest after the meal and the time of occurrence of the first minimum extreme value AI_{P1}. The electronic device 1 of this embodiment can further estimate the state of lipid metabolism of the subject on the basis of the second minimum extreme value AI_{P2} detected after a predetermined time or longer after the detection of the first minimum extreme value AI_{P1} and the time of occurrence of the second minimum extreme value AI_{P2}.

In this embodiment, triglyceride has been described as an example estimation of lipid metabolism, the estimation of lipid metabolism is not limited to triglyceride. The lipid value estimated by the electronic device 1 includes, for example, total cholesterol, good (HDL: High Density Lipoprotein) cholesterol, bad (LDL: Low Density Lipoprotein) cholesterol, and the like. These lipid values also exhibit tendencies similar to that for triglyceride described above.

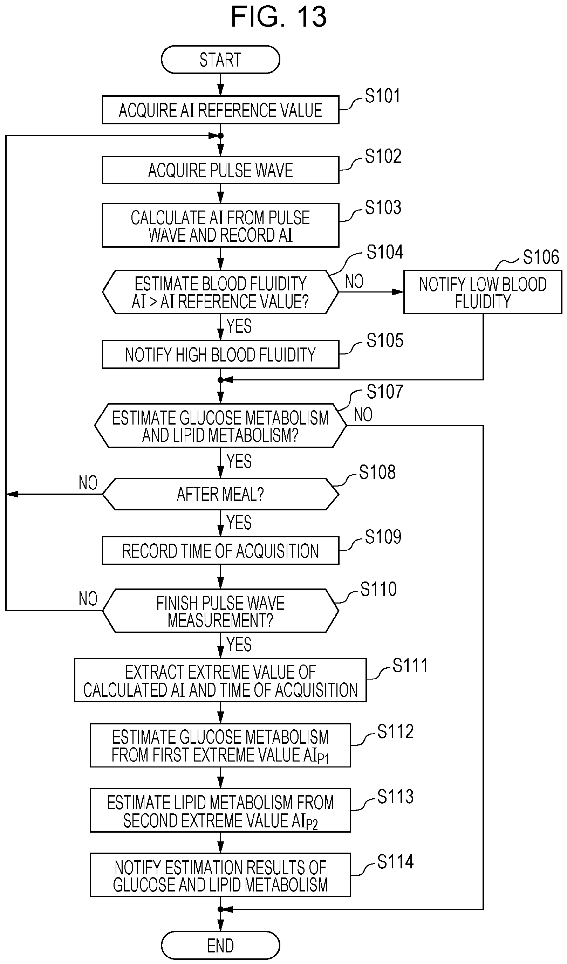

Fig. 13 is a flowchart illustrating a procedure for estimating blood fluidity and the states of glucose metabolism and lipid metabolism on the basis of the AI. Referring to Fig. 13, the flow of estimation of blood fluidity and the states of glucose metabolism and lipid metabolism using the electronic device 1 according to the embodiment will be described.

As illustrated in Fig. 13, the electronic device 1 acquires an AI reference value of the subject as an initial setting (step S101). The AI reference value may be implemented using an average AI estimated from the age of the subject or the AI of the subject on an empty stomach that is acquired in advance. The electronic device 1 may set the AI determined to be before the meal in steps S102 to S108 as the AI reference value, or may set the AI calculated immediately before pulse wave measurement as the AI reference value. In this case, the electronic device 1 executes step S101 after steps S102 to S108.

Then, the electronic device 1 acquires a pulse wave (step S102). For example, the electronic device 1 determines whether a predetermined amplitude or more is obtained for a pulse wave acquired for a predetermined measurement time (for example, 5 seconds). If the predetermined amplitude or more is obtained for the acquired pulse wave, the process proceeds to step S103. If the predetermined amplitude or more is not obtained, step S102 is repeatedly performed (these steps are not illustrated). For example, upon detecting a pulse wave having the predetermined amplitude or more in step S102, the electronic device 1 automatically acquires the pulse wave.

The electronic device 1 calculates, from the pulse wave acquired in step S102, an AI as an index based on the pulse wave and stores the AI in the storage unit 54 (step S103). The electronic device 1 may calculate an average value AIₐᵥₑ from the AIₙ (n is an integer of 1 to n) every predetermined pulse rate (for example, three pulses), and set the average value AIₐᵥₑ as the AI. Alternatively, the electronic device 1 may calculate the AI at a specific pulse.

The AI may be calculated by, for example, performing correction based on a pulse rate P_{R}, a pulse pressure (P_{F} - P_{S}), a body temperature, the temperature of the detected portion, and so on. It is known that there is a negative correlation between the pulse and the AI and between the pulse pressure and the AI and that there is a positive correlation between the temperature and the AI. In order to perform correction, for example, in step S103, the electronic device 1 calculates the pulse and the pulse pressure in addition to the AI. For example, the sensor 50 may include a temperature sensor, and the electronic device 1 may acquire the temperature of the detected portion when acquiring the pulse wave in step S102. The AI is corrected by substituting the acquired pulse, pulse pressure, temperature, and so on into a correction formula created in advance.

Then, the electronic device 1 compares the AI reference value acquired in step S101 with the AI calculated in step S103 and estimates the blood fluidity of the subject (step S104). If the calculated AI is larger than the AI reference value (in the case of YES), it is estimated that the blood fluidity is high, and the electronic device 1 notifies the subject that, for example, the blood fluidity is high (step S105). If the calculated AI is not larger than the AI reference value (in the case of NO), it is estimated that the blood fluidity is low, and the electronic device 1 notifies the subject that, for example, the blood fluidity is low (step S106).

Then, the electronic device 1 asks the subject whether to estimate the states of glucose metabolism and lipid metabolism (step S107). If none of glucose metabolism and lipid metabolism is to be estimated in step S107 (in the case of NO), the electronic device 1 ends the process. If glucose metabolism and lipid metabolism are to be estimated in step S107 (in the case of YES), the electronic device 1 asks the subject whether the calculated AI is acquired before or after the meal (step S108). If the calculated AI is not acquired after the meal (the calculated AI is acquired before the meal) (in the case of NO), the process returns to step S102, and the next pulse wave is acquired. If the calculated AI is acquired after the meal (in the case of YES), the electronic device 1 stores the time at which the pulse wave corresponding to the calculated AI is acquired (step S109). If the pulse wave is to be continuously acquired (in the case of NO in step S110), the process returns to step S102, and the next pulse wave is acquired. If the pulse wave measurement is to be finished (in the case of YES in step S110), the process proceeds to step S111 and the subsequent steps, and the electronic device 1 estimates the states of glucose metabolism and lipid metabolism of the subject.

Then, the electronic device 1 extracts a minimum extreme value and the time thereof from a plurality of AIs calculated in step S104 (step S111). For example, if the AI indicated by the solid line in Fig. 12 is calculated, the electronic device 1 extracts the first minimum extreme value AI_{P1} at about 30 minutes after the meal and the second minimum extreme value AI_{P2} at about 2 hours after the meal.

Then, the electronic device 1 estimates the state of glucose metabolism of the subject from the first minimum extreme value AI_{P1} and the time thereof (step S112). The electronic device 1 further estimates the state of lipid metabolism of the subject from the second minimum extreme value AI_{P2} and the time thereof (step S113). An example estimation of the states of glucose metabolism and lipid metabolism of the subject is similar to that in Fig. 12 described above and will not thus described.

Then, the electronic device 1 notifies the subject of the estimation results obtained in step S112 and step S113 (step S114), and then ends the process illustrated in Fig. 13. The notification unit 20 provides a notification such as "glucose metabolism is normal", "glucose metabolism abnormality is suspected", "lipid metabolism is normal", or "lipid metabolism abnormality is suspected". In this case, the notification unit 20 may provide the notification described above by, for example, turning on or blinking the light-emitting unit. Alternatively, the notification unit 20 may notify the subject of advice such as "You are advised to visit the hospital" or "You are advised to improve your diet". Then, the electronic device 1 ends the process illustrated in Fig. 13.

In this embodiment, the electronic device 1 can estimate the blood fluidity and the states of glucose metabolism and lipid metabolism of the subject from the indices based on the pulse wave. Accordingly, the electronic device 1 can estimate the blood fluidity and the states of glucose metabolism and lipid metabolism of the subject in a noninvasive manner and in a short time.

In this embodiment, the electronic device 1 can perform the estimation of the state of glucose metabolism and the estimation of the state of lipid metabolism from extreme values of an index based on the pulse wave and the times thereof. Accordingly, the electronic device 1 can estimate the states of glucose metabolism and lipid metabolism of the subject in a noninvasive manner and in a short time.

In this embodiment, the electronic device 1 can estimate the states of glucose metabolism and lipid metabolism of the subject on the basis of, for example, the index based on the pulse wave before the meal (on an empty stomach). Accordingly, it is possible to accurately estimate the blood fluidity and the states of glucose metabolism and lipid metabolism of the subject without taking into consideration the blood vessel diameter, the vascular stiffness, or the like, which does not change in a short time.

In this embodiment, the electronic device 1 performs calibration between the index based on the pulse wave and the blood glucose level and between the index based on the pulse wave and the lipid value, thereby being capable of estimating the blood glucose level and the lipid value of the subject in a noninvasive manner and in a short time.

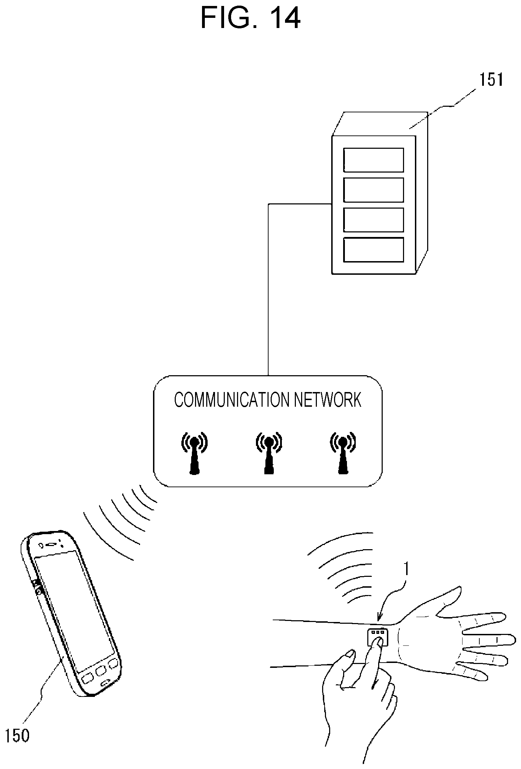

Fig. 14 is a schematic diagram illustrating a schematic configuration of a system according to the first embodiment. The system illustrated in Fig. 14 is configured to include the electronic device 1, a server 151, a mobile terminal 150, and a communication network. As illustrated in Fig. 14, an index based on the pulse wave calculated by the electronic device 1 is transmitted to the server 151 via the communication network and is saved in the server 151 as personal information of the subject. The server 151 compares the index based on the pulse wave with previously acquired information of the subject and/or various databases to estimate the blood fluidity and the states of glucose metabolism and lipid metabolism of the subject. The server 151 further creates optimum advice for the subject. The server 151 returns the estimation results and the advice to the mobile terminal 150 possessed by the subject. The mobile terminal 150 notifies the subject of the received estimation results and advice through a display unit of the mobile terminal 150. Such a system can be constructed. Using the communication function of the electronic device 1 enables the server 151 to collect information from a plurality of users, resulting in a further increase in the accuracy of estimation. Since the mobile terminal 150 is used as a notification means, the electronic device 1 no longer requires the notification unit 20 and is further reduced in size. Further, since the server 151 estimates the blood fluidity and the states of glucose metabolism and lipid metabolism of the subject, the computational load on the control unit 52 of the electronic device 1 can be reduced. Further, since previously acquired information of the subject can be saved in the server 151, the load on the storage unit 54 of the electronic device 1 can be reduced. This results in further reduction in the size and complexity of the electronic device 1. In addition, the computational processing speed is also improved.

While the configuration of the system according to this embodiment has been described in which the electronic device 1 and the mobile terminal 150 are connected to each other via the server 151 over the communication network, a system according to the present invention is not limited to this. The electronic device 1 and the mobile terminal 150 may be directly connected to each other over the communication network without using the server 151.

Characteristic examples have been described to fully and clearly disclose the present disclosure. However, the appended claims are not to be limited to the embodiment described above, but are to be configured to embody all modifications and alternative configurations that may be created by a person skilled in the art in this technical field within the scope of the basic matter described herein.

For example, in the embodiment described above, a case has been described in which an angular speed sensor is provided as the sensor 50. However, the form of the electronic device 1 is not limited to this. The sensor 50 may include an optical pulse wave sensor including a light-emitting unit and a light-receiving unit, or may include a pressure sensor. In addition, the target region to be subjected to measurement of biological information by the electronic device 1 is not limited to the wrist of the subject. It is sufficient that the sensor 50 be placed over an artery, such as in a neck, an ankle, a thigh, or an ear.

For example, in the embodiment described above, the states of glucose metabolism and lipid metabolism of the subject are estimated on the basis of the first extreme value and the second extreme value of the index based on the pulse wave and the times thereof. However, the processing executed by the electronic device 1 is not limited to this. In some cases, only either extreme value may appear, or no extreme value may appear. The electronic device 1 may estimate the states of glucose metabolism and lipid metabolism of the subject on the basis of the overall tendency (for example, an integral value, Fourier transform, etc.) of the time variation in the index based on the calculated pulse wave. The electronic device 1 may estimate the states of glucose metabolism and lipid metabolism of the subject on the basis of a time range in which the index based on the pulse wave is less than or equal to a predetermined value, instead of by extracting extreme values of the index based on the pulse wave.

For example, in the embodiment described above, a case has been described in which the blood fluidity before and after a meal is estimated. However, the processing executed by the electronic device 1 is not limited to this. The electronic device 1 may estimate the blood fluidity before and after exercise and during exercise, or may estimate the blood fluidity before and after bathing and during bathing.

In the embodiment described above, the electronic device 1 measures the pulse wave. However, the pulse wave may not necessarily be measured by the electronic device 1. For example, the electronic device 1 may be connected to an information processing device such as a computer or a mobile phone in a wired or wireless manner, and angular speed information acquired by the sensor 50 may be transmitted to the information processing device. In this case, the information processing device may measure the pulse wave on the basis of the angular speed information. The information processing device may execute processing for estimating glucose metabolism and lipid metabolism, or the like. In a case where the information processing device connected to the electronic device 1 executes various types of information processing, the electronic device 1 may not include the control unit 52, the storage unit 54, the notification unit 20, or the like. In a case where the electronic device 1 is connected to the information processing device in a wired manner, the electronic device 1 may not include the battery 60 and may be supplied with power from the information processing device.

In the embodiment described above, an example has been described in which the electronic device 1 is constituted by the lower housing 11 and the upper housing 12 each having a rectangular shape. However, the shape of the housing of the electronic device 1 may not be a rectangular shape. For example, in one embodiment, the electronic device 1 may be constituted by the lower housing 11 and the upper housing 12 each having a shape such as a disk or a triangle. In one embodiment, the electronic device 1 may have various configurations such that the sensor 50 is pressed toward the target region through the elastic member 70.

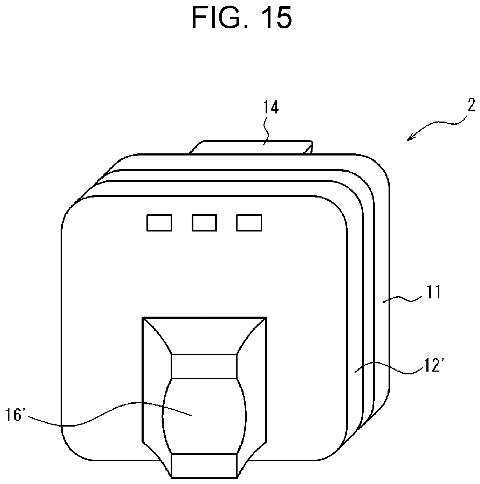

The pressing portion 16 included in the upper housing 12 of the electronic device 1 may also have various configurations. For example, as in an electronic device 2 illustrated in Fig. 15, a knob-shaped pressing portion 16' may be included. The pressing portion 16' having the shape illustrated in Fig. 15 allows the subject to hold the pressing portion 16' with, for example, a convex portion thereof pinched between the thumb and the index finger or the like to position the electronic device 1 with respect to the target region at the start of measurement. After positioning the electronic device 1, the subject can maintain the state in which the convex portion of the pressing portion 16' is held between the thumb and the index finger or the like, or may press the concave portion of the pressing portion 16'.

The control unit 52 of the electronic device 1 may estimate at least any one of glucose and lipid metabolism, blood glucose level, and lipid value from the index of the pulse wave. The electronic device 1 may function as a diet monitor that monitors the progress of a diet of the subject or a blood glucose meter that monitors the blood glucose level of the subject.

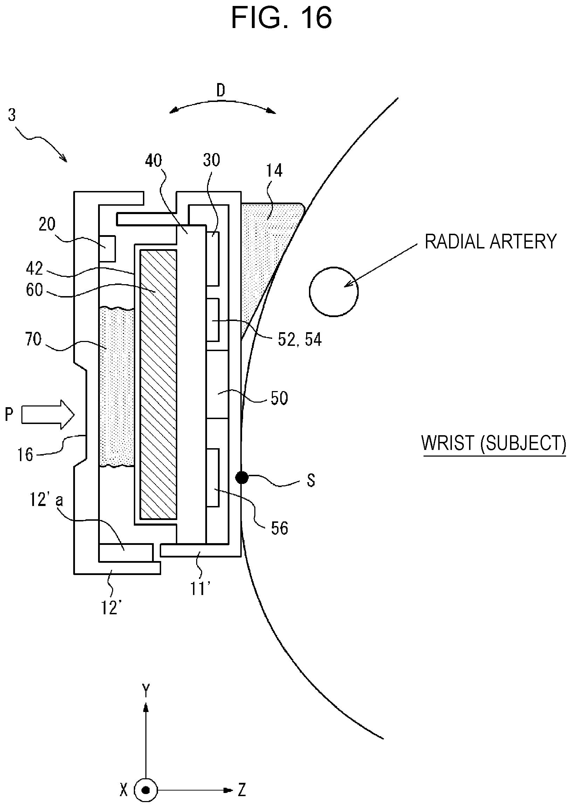

A modification of the electronic device according to the embodiment described above will further be described. Fig. 16 is a diagram illustrating a cross section of a modification of the electronic device according to the embodiment described above. As illustrated in Fig. 16, an electronic device 3 includes a housing 12' and a housing 11' to be nested together. In the electronic device 3, pressing the housing 12' against the housing 11' deforms the elastic member 70, and brings a protrusion 12'a into contact with the housing 11'. Then, the housing 11' rotates about the protrusion 12'a as a fulcrum. That is, in the electronic device 3, the protrusion 12'a acting as a predetermined rotary axis is located near an end of the upper housing 12' provided with the pressing portion 16 and near an end of the lower housing 11' including the sensor 50.

### Reference Signs List

- 1, 2, 3: electronic device
- 11, 11': lower housing
- 12, 12': upper housing
- 12'a: protrusion
- 14: pulse contact portion
- 16: pressing portion
- 20: notification unit
- 30: switch
- 40: substrate
- 42: battery holder
- 50: sensor
- 52: control unit
- 54: storage unit
- 56: communication unit
- 60: battery
- 70: elastic member
- 150: mobile terminal
- 151: server

## Claims

1. An electronic device comprising:
a sensor capable of detecting pulsation in a target region of a subject;
a pressing portion to be pressed toward the target region; and
an elastic member interposed between the sensor and the pressing portion.

2. The electronic device according to Claim 1, wherein the elastic member is deformable in accordance with the pulsation in the target region.

3. The electronic device according to Claim 1 or 2,
wherein the elastic member is three-dimensionally deformable.

4. The electronic device according to any of Claims 1 to 3, wherein the elastic member is elastically deformed to such an extent that the pulsation in the target region is detectable by the sensor.

5. The electronic device according to any of Claims 1 to 4, wherein the sensor detects the pulsation in the target region as a portion of a rotational movement about a predetermined axis.

6. The electronic device according to Claim 5, wherein the sensor detects the pulsation in the target region as rotational movements on at least two axes.

7. The electronic device according to Claim 6, wherein the sensor detects the pulsation in the target region as rotational movements on three axes.

8. The electronic device according to any of Claims 1 to 7, wherein the sensor is a gyro sensor.

9. The electronic device according to any of Claims 1 to 8, further comprising
a control unit that calculates an index of a pulse wave based on the pulsation detected by the sensor, wherein
the control unit combines results detected by the sensor as rotational movements on at least two axes.

10. The electronic device according to Claim 9, wherein the control unit combines only results having components greater than or equal to a predetermined threshold among the results detected by the sensor as rotational movements on at least two axes.

11. The electronic device according to Claim 9 or 10,
wherein the control unit combines the results detected by the sensor as rotational movements on at least two axes after polarities of the results are made to match each other.

12. The electronic device according to any of Claims 1 to 11, wherein the elastic member is an elastic member deformable along at least any one axis among three axes orthogonal to one another.

13. The electronic device according to any of Claims 1 to 12, wherein the control unit estimates at least any one of glucose and lipid metabolism, a blood glucose level, and a lipid value from the index of the pulse wave.

14. The electronic device according to any of Claims 1 to 13, wherein the electronic device functions as a diet monitor that monitors a progress of a diet of the subject or a blood glucose meter that monitors a blood glucose level of the subject.

15. The electronic device according to any of Claims 1 to 14, wherein the electronic device has a size such that the electronic device is housed in a square with sides, each of which is up to 4 cm, in plan view.

16. The electronic device according to any of Claims 1 to 15, further comprising a first housing and a second housing to be nested together, the first housing including a protrusion that comes into contact with the second housing.
